# EUROPEAN PATENT APPLICATION

(11) **EP 4 497 405 A2**
(43) Date of publication of application: **29.01.2025**
(21) Application number: 24191039.7
(22) Date of filing: 26.07.2024
(51) Int. Cl.: A61B 18/14, A61B 17/00, A61B 18/00

(54) **DOUBLE-SIDED ENCAPSULATED PLANAR CATHETER WITH ENCAPSULATING POLYMER**

(30) Priority: 28.07.2023 US 202363516196 P; 28.06.2024 US 202418758612
(71) Applicant: Biosense Webster (Israel) Ltd., 2066717 Yokneam (IL)
(72) Inventor: HENRIQUEZ, Jamie, Irvine, 92618 (US); GOO, Audrey Claire, Irvine, 92618 (US); ABBAS, Mohammad, Irvine, 92618 (US); RODRIGUEZ SOTO, Juan, Irvine, 92618 (US); VAN NIEKERK, Pieter Emmelius, Irvine, 92618 (US); EBRAHIMI, Babak, Irvine, 92618 (US)
(74) Representative: Carpmaels & Ransford LLP

(57) **Abstract**

An end effector for a cardiac mapping catheter including a flexible circuit including a plurality of electrodes each electrode of the plurality of electrodes including a contact surface. The flexible circuit can be disposed on an insulative material. The insulative material can be contiguous to the contact surfaces so that only the contact surfaces of at least a portion of the plurality of electrodes are exposed to the ambient environment.

## Description

This application claims priority to prior filed U.S. Patent Application No. 63/516,196 filed on July 28, 2023, Attorney Docket No. 253757.000421 (BIO6876USPSP1), which is hereby incorporated by reference as set forth in full herein.

### FIELD

The present invention relates generally to minimally invasive medical devices, and in particular sensing catheters, and further relates to, but not exclusively, cardiac mapping catheters and the manufacture thereof.

### BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals can be located in tissue of an atria or a ventricle. Unwanted signals are conducted elsewhere through heart tissue where they can initiate or continue arrhythmia.

Procedures for treating arrhythmia include surgically disrupting the origin of the signals causing the arrhythmia, as well as disrupting the conducting pathway for such signals. More recently, it has been found that by mapping the electrical properties of the endocardium and the heart volume, and selectively ablating cardiac tissue by application of energy, it is possible to cease or modify the propagation of unwanted electrical signals from one portion of the heart to another. The ablation process destroys the unwanted electrical pathways by formation of non-conducting lesions.

In this two-step procedure, which includes mapping followed by ablation, electrical activity at points in the heart is typically sensed and measured by advancing a catheter containing one or more electrical sensors into the heart and acquiring data at multiple points. These data are then utilized to select the target areas at which ablation is to be performed.

For greater mapping resolution, it is desirable for a mapping catheter to conform closely to the target anatomy. For mapping within an atria or a ventricle (for example, an apex of a ventricle), it is desirable for a catheter to collect larger amounts of data signals within shorter time spans. It is also desirable for such a catheter to be capable of allowing sufficient electrode contact with different tissue surfaces, for example, flat, curved, irregular or nonplanar surface tissue, and be collapsible for atraumatic advancement and withdrawal through a patient's vasculature. Existing catheters generally require stiff internal structural members to ensure that a predetermined configuration is maintained. The stiffness is a disadvantage during manipulation in the body organ as it can prevent electrodes from contacting the tissue.

Other catheters can include flexible end effectors designed to overcome this disadvantage. These catheters can include layered components that can be time-consuming, complex, and expensive to manufacture and assemble. Many times, electrode contact surfaces of these end effectors must be exposed through an encapsulating material via laser cutting or mechanical removal, both of which increase labor time and production costs.

### SUMMARY

To enable the time- and cost-efficient manufacture of end effectors that enhance the function of mapping and ablation catheters mentioned in the background section, the present disclosure relates to easily manufacturable end effectors, as well as fixtures and methods for manufacturing said end effectors having enhanced aspects of mapping and ablation catheter performance, including, but not limited to: mapping resolution, electrode contact with the target anatomy, delivery of the end effector to the target anatomy, biocompatibility, end effector stiffness, and atraumaticity.

There is provided, in accordance with an embodiment of the present invention, an end effector. The end effector can include a flexible circuit including a plurality of electrodes each electrode of the plurality of electrodes including a contact surface. The flexible circuit can be disposed on an insulative material. The insulative material can be contiguous to the contact surfaces so that only the contact surfaces of at least a portion of the plurality of electrodes are exposed to the ambient environment.

The disclosed technology includes a method of manufacturing an end effector for a medical catheter. The method can include placing a flexible circuit including a plurality of electrodes in a mold, each electrode of the plurality of electrodes including a contact surface, placing a sheet of insulative material in contact with the flexible circuit, and molding the flexible circuit and the insulative material to cause the insulative material to flow at least partially around the flexible circuit contiguous to the contact surface so that only the contact surface of at least a portion of the electrodes of the plurality of electrodes is exposed to the ambient environment.

The disclosed technology includes a fixture for forming an encapsulated end effector for a medical catheter. The fixture can include a plurality of components stacked generally parallel along a vertical axis of the fixture. The plurality of components can include a first compression surface, a second compression surface disposed opposite the first compression surface and configured to translate along the vertical axis with respect to the first compression surface from an expanded configuration to a compressed configuration, and a first flexible heat resistant sheet disposed proximate the first compression surface and having a durometer such that contact surfaces of a first plurality of electrodes extending from a first flexible circuit protrude into the first flexible heat resistant sheet a first distance upon applying compression to the first compression surface and the second compression surface.

### BRIEF DESCRIPTION OF THE DRAWINGS

FIG. 1 is a schematic pictorial illustration of a medical system including a planar catheter, in accordance with an embodiment of the present invention;
FIG. 2 is an illustration of a perspective view of an end effector, in accordance with an embodiment of the present invention;
FIG. 3 is an illustration of an exploded perspective view of the end effector of FIG. 2;
FIG. 4A is an illustration of a cross section of an end effector, in accordance with an embodiment of the present invention;
FIG. 4B is an illustration of a cross section of an end effector, in accordance with an embodiment of the present invention;

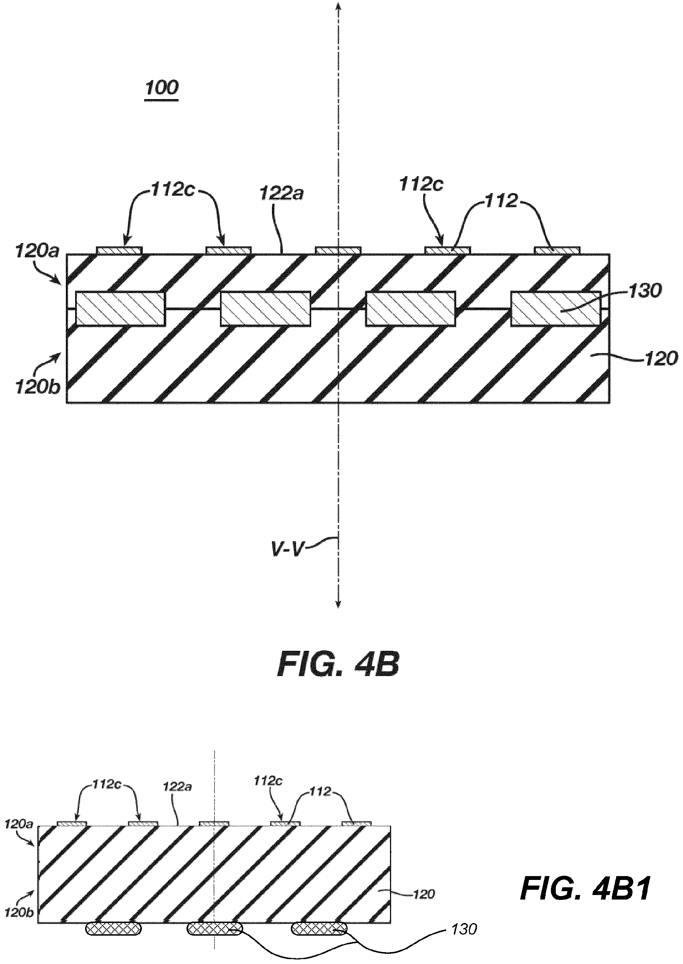

FIG. 4B 1 is a variation of Fig. 4B in which a framework is not encapsulated into the insulative material but is contiguous therewith;

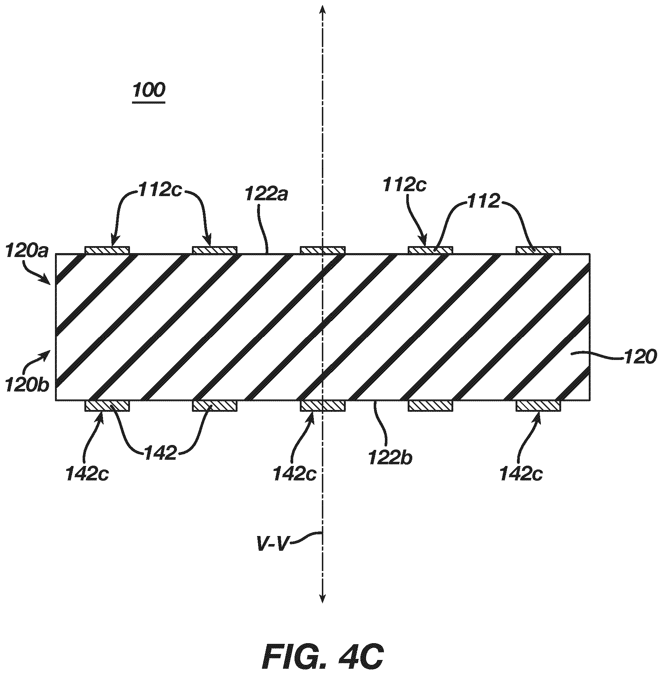

FIG. 4C is an illustration of a cross section of an end effector, in accordance with an embodiment of the present invention;

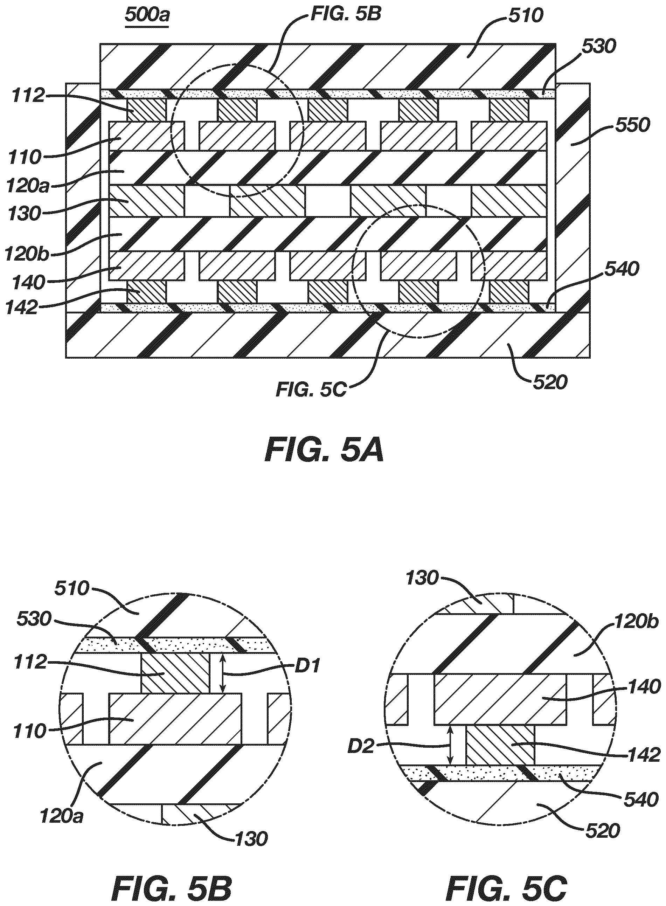

FIG. 5A is an illustration of a cross section of a fixture for making an end effector, in accordance with an embodiment of the present invention;
FIG. 5B is a detail view of a cross section of a fixture for making an end effector, in accordance with an embodiment of the present invention;
FIG. 5C is a detail view of a cross section of a fixture for making an end effector, in accordance with an embodiment of the present invention;

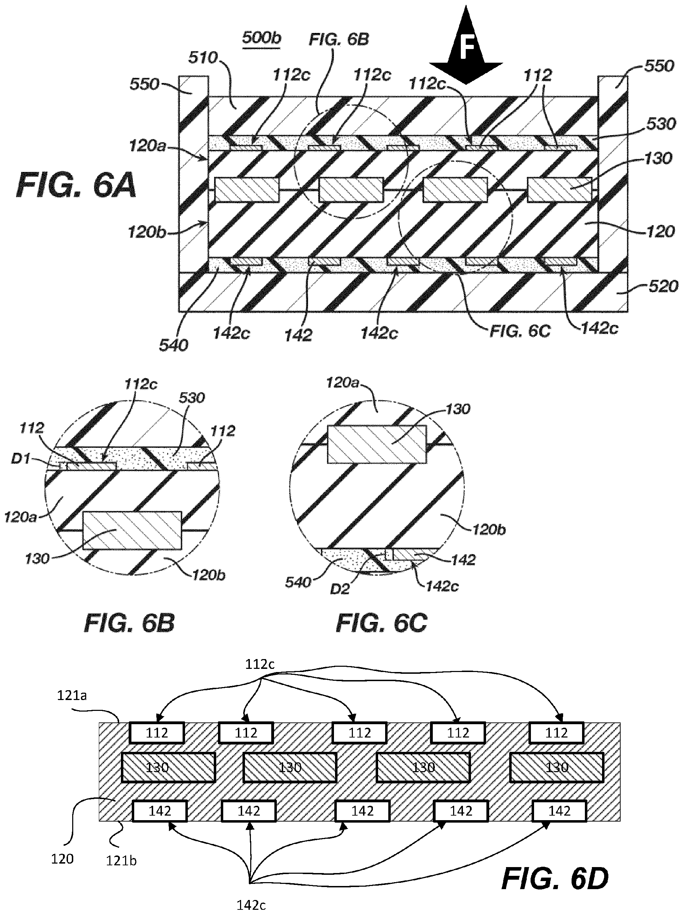

FIG. 6A is an illustration of a cross section of a fixture for making an end effector, in accordance with an embodiment of the present invention;
FIG. 6B is a detail view of a cross section of a fixture for making an end effector, in accordance with an embodiment of the present invention;
FIG. 6C is a detail view of a cross section of a fixture for making an end effector, in accordance with an embodiment of the present invention;
FIG. 6D shows a cross-sectional view of the end effector removed from the fixture showing the electrode contact surfaces flush with the insulative substrate, in accordance with an embodiment of the present invention;

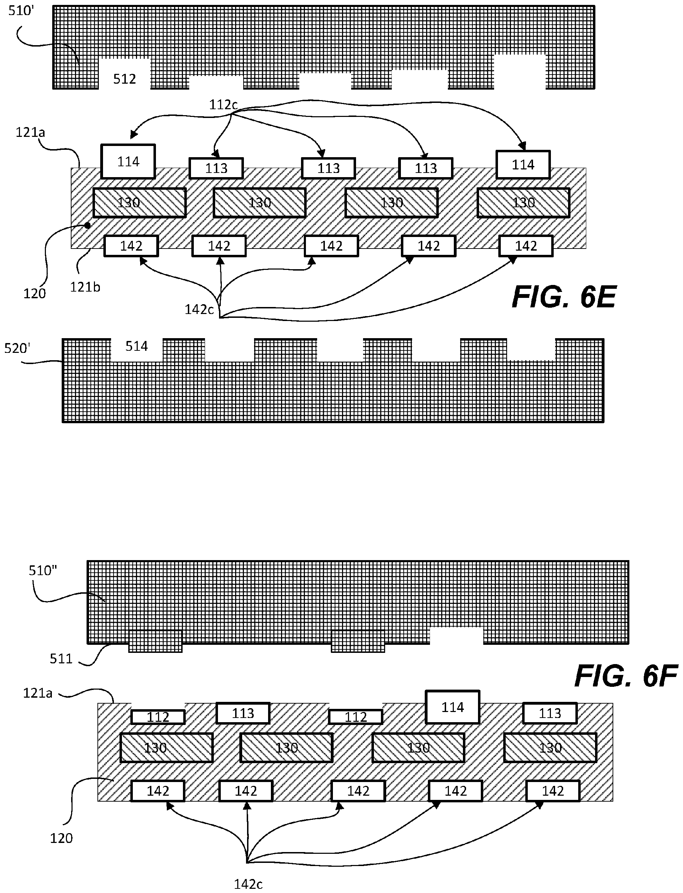

FIGS. 6E and 6F show alternative embodiments where the electrode contact surfaces do not have to be flush with the insulative surface and can be varied, in accordance with embodiments of the present invention;

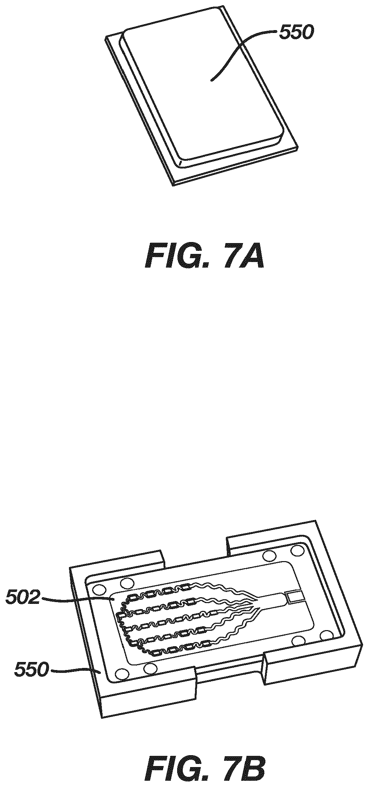

FIG. 7A is an illustration of a perspective view of a fixture for making an end effector, in accordance with an embodiment of the present invention;
FIG. 7B is an illustration of a perspective view of a fixture for making an end effector, in accordance with an embodiment of the present invention;

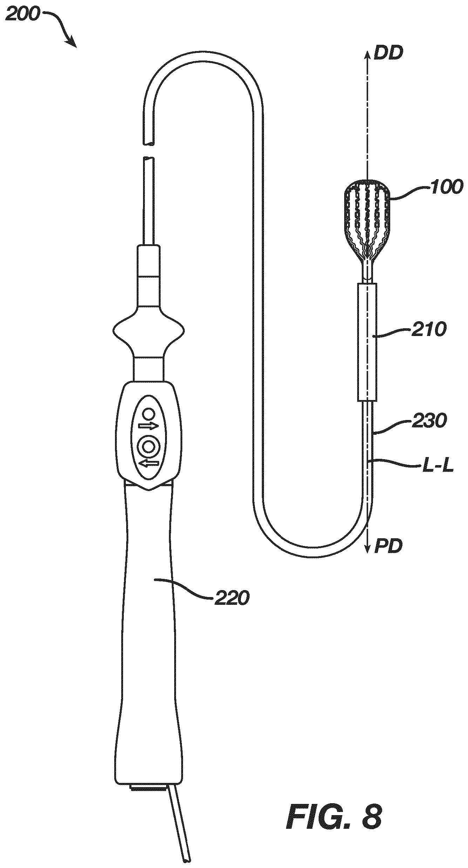

FIG. 8 is an illustration of a catheter assembly, in accordance with an embodiment of the present invention

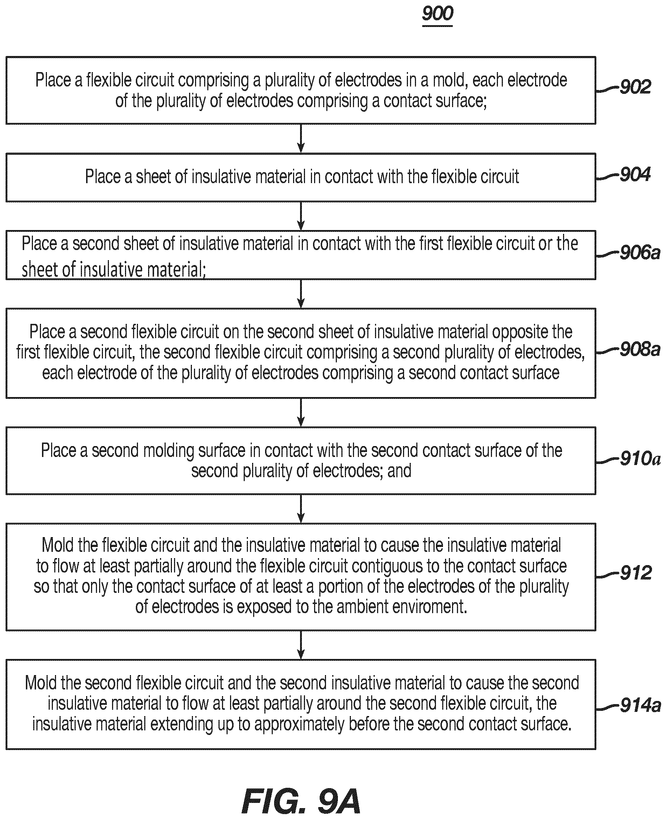

FIG. 9A is a flowchart of a method of manufacturing an end effector for a medical catheter, in accordance with an example of the present invention; and

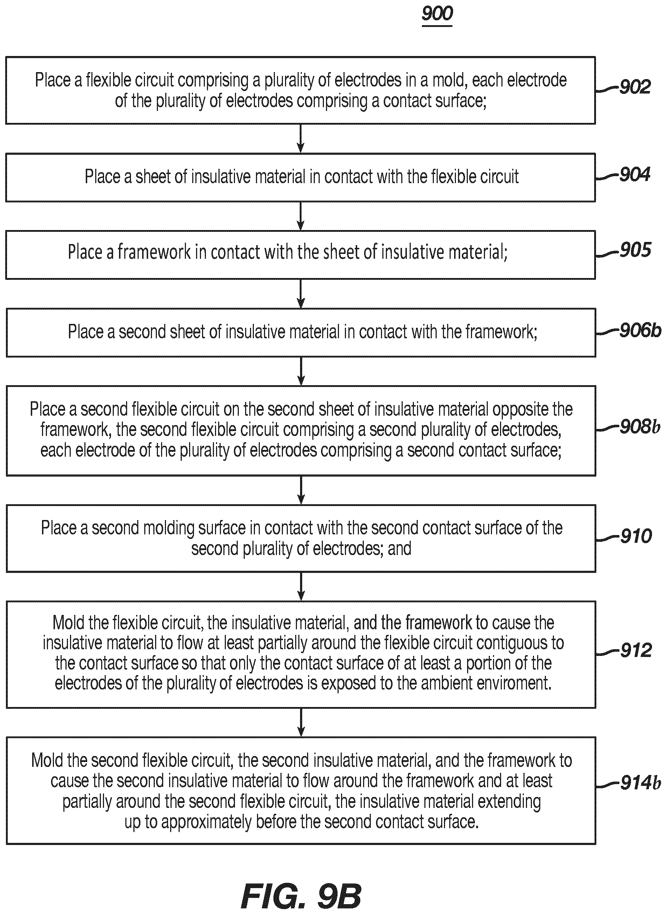

FIG. 9B is a flowchart of a method of manufacturing an end effector for a medical catheter, in accordance with an example of the present invention.

### DETAILED DESCRIPTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, reference to tissue, vasculature, or organs of a "patient," "host," "user," and "subject" can be that of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells. For example, by utilizing thermal energy, such as radio frequency (RF) ablation, or non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

The present disclosure is related to systems, methods or uses and devices for mapping and ablation of cardiac tissue to treat cardiac arrhythmias. Ablative energies are typically provided to cardiac tissue by a tip portion of a catheter which can deliver ablative energy alongside the tissue to be ablated. Some example catheters include three-dimensional structures at the tip portion and are configured to administer ablative energy from various electrodes positioned on the three-dimensional structures. Ablative procedures incorporating such example catheters can be visualized using fluoroscopy.

Ablation of cardiac tissue using application of a thermal technique, such as radio frequency (RF) energy and cryoablation, to correct a malfunctioning heart is a well-known procedure. Typically, to successfully ablate using a thermal technique, cardiac electropotentials need to be measured at various locations of the myocardium. In addition, temperature measurements during ablation provide data enabling the efficacy of the ablation. Typically, for an ablation procedure using a thermal technique, the electropotentials and the temperatures are measured before, during, and after the actual ablation. RF approaches can have risks that can lead to tissue charring, burning, steam pop, phrenic nerve palsy, pulmonary vein stenosis, and esophageal fistula. Cryoablation is an alternative approach to RF ablation that can reduce some thermal risks associated with RF ablation. However maneuvering cryoablation devices and selectively applying cryoablation is generally more challenging compared to RF ablation; therefore, cryoablation is not viable in certain anatomical geometries which may be reached by electrical ablation devices.

The present disclosure can include electrodes configured for RF ablation, cryoablation, and/or irreversible electroporation (IRE). IRE can be referred to throughout this disclosure interchangeably as pulsed electric field (PEF) ablation and pulsed field ablation (PFA). IRE as discussed in this disclosure is a non-thermal cell death technology that can be used for ablation of atrial arrhythmias. To ablate using IRE/PEF, biphasic voltage pulses are applied to disrupt cellular structures of myocardium. The biphasic pulses are non-sinusoidal and can be tuned to target cells based on electrophysiology of the cells. In contrast, to ablate using RF, a sinusoidal voltage waveform is applied to produce heat at the treatment area, indiscriminately heating all cells in the treatment area. IRE therefore has the capability to spare adjacent heat sensitive structures or tissues which would be of benefit in the reduction of possible complications known with ablation or isolation modalities. Additionally, or alternatively, monophasic pulses can be utilized.

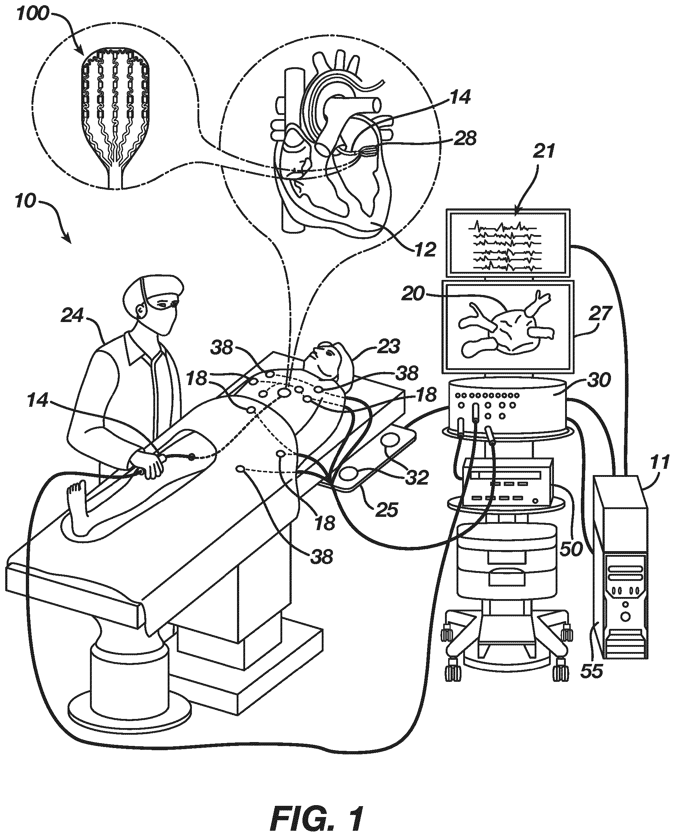

Reference is made to FIG. 1 showing an example catheter-based electrophysiology mapping and ablation system 10. System 10 includes multiple catheters, which are percutaneously inserted by physician 24 through the patient's 23 vascular system into a chamber or vascular structure of a heart 12. Typically, a delivery sheath catheter is inserted into the left or right atrium near a desired location in heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. Physician 24 brings a catheter shaft 90 with distal tip of catheter 14 (i.e., multilayered end effector 100) into contact with the heart wall for sensing a target site in heart 12. For ablation, physician 24 would similarly bring a distal end of an ablation catheter to a target site for ablating.

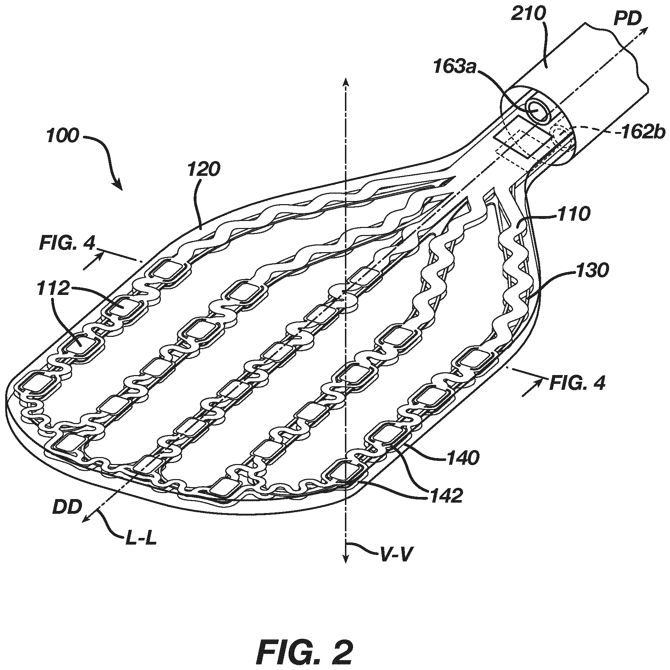

Catheter 14 is an exemplary catheter that includes one and preferably multiple electrodes 26 optionally distributed over end effector 100 coupled to a catheter shaft 90 and configured to sense the IEGM signals as described in more detail below. Catheter 14 may additionally include a position sensor (as shown in FIG. 2B) embedded in or near end effector 100 for tracking position and orientation of end effector 100. Optionally and preferably, position sensor is a magnetic based position sensor including multiple magnetic coils for sensing three-dimensional (3D) position and orientation.

Magnetic based position sensor may be operated together with a location pad 25 including a plurality of magnetic coils 32 configured to generate magnetic fields in a predefined working volume. Real time position of end effector 100 of catheter 14 may be tracked based on magnetic fields generated with location pad 25 and sensed by magnetic based position sensor. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System 10 includes one or more electrode patches 38 positioned for skin contact on patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182, each of which are incorporated herein by reference.

A recorder 11 displays electrograms 21 captured with body surface ECG electrodes 18 and intracardiac electrograms (IEGM) captured with electrodes 26 of catheter 14. Recorder 11 may include pacing capability for pacing the heart rhythm and/or may be electrically connected to a standalone pacer.

System 10 may include an ablation energy generator 50 that is adapted to conduct ablative energy to one or more of electrodes 26 at a distal tip of a catheter configured for ablating. Energy produced by ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) 30 is an interface configured to establish electrical communication between catheters, electrophysiological equipment, power supply and a workstation 55 for controlling operation of system 10. Electrophysiological equipment of system 10 may include for example, multiple catheters, location pad 25, body surface ECG electrodes 18, electrode patches 38, ablation energy generator 50, and recorder 11. Optionally and preferably, PIU 30 additionally includes processing capability for implementing real-time computations of location of the catheters and for performing ECG calculations.

Workstation 55 includes memory, processor unit with memory or storage with appropriate operating software loaded therein, and user interface capability. Workstation 55 may provide multiple functions, optionally including (1) modeling the endocardial anatomy in three-dimensions (3D) and rendering the model or anatomical map 20 for display on a display device 27, (2) displaying on display device 27 activation sequences (or other data) compiled from recorded electrograms 21 in representative visual indicia or imagery superimposed on the rendered anatomical map 20, (3) displaying real-time location and orientation of multiple catheters within the heart chamber, and (5) displaying on display device 27 sites of interest such as places where ablation energy has been applied. One commercial product embodying elements of the system 10 is available as the CARTO^{™} 3 System, available from Biosense Webster, Inc., 31 Technology Drive, Suite 200, Irvine, CA 92618.

FIG. 2 provides an end effector 100 in accordance with an embodiment of the present disclosure in order to achieve the ease of manufacturing, reduced cost, and enhanced end effector properties, such as desired stiffness, mapping resolution, electrode contact with target anatomy, and conformity of the end effector disclosed herein to flat, curved, irregular and/or nonplanar tissue surfaces found in the target anatomy. The end effector 100 can include a flexible circuit 110 including a plurality of electrodes 112, each electrode of the plurality of electrodes 112 including a contact surface 112c. As used herein, the term "flexible circuit" includes thin-film circuit, flexible printed circuit board, thin film deposition via lithography and etching processes on substrate such as polyimide, copper, LCP (e.g., Panasonic FELIOS^{™} SERIES of Flexible Circuit Boards), nitinol substrate or directly onto the polymeric substrate such as Panasonic BEYOLEX^{™} PRINTED ELECTRONICS SUBSTRATE Series as shown and described in the attached technical references incorporated herein by the Appendix. In some examples, the flexible circuits described herein can be made primarily of polyimide. In other examples, it can be made of any of biocompatible polyimides, glass-reinforced epoxy laminate materials, copper, or graphene, alone or in combination. The first 110 and second 140 flexible circuits can include conductive traces directly formed onto the polymeric substrate with the electrode comprising a substantially much thicker version of the traces such that the typical polyimide substrate is no longer required. In some examples, the electrodes described herein can include at least one mapping electrode and/or at least one ablation electrode and can be configured to detect electrophysiological signals or transmit ablative energy AC or DC from an energy generator to the tissue according to the various ablation methods previously described e.g., RF, IRE, etc.

The flexible circuit 110 can be disposed on an insulative material 120. The insulative material 120 can be contiguous to the contact surfaces 112c so that only the contact surfaces 112c of at least a portion of the plurality of electrodes 112 are exposed to the ambient environment. As used herein, the term "contact surface" includes the portion of an electrode having a generally flat surface and the edge or edges which immediately surround said flat surface. Electrodes 112 may have a slightly rounded, radiused, or chamfered edge that comes into contact with tissue, along with the generally flat surface, when the end effector 100 is placed against tissue. As used herein, "ambient environment" refers to the external environment such as the organ in which the end effector 100 is deployed or in the operating theater prior to being deployed in the biological organ.

It is noted that not all of the electrodes on the end effector 100 described herein need be exposed through the insulative material 120 as these non-exposed electrodes can be used to sense far-field signals for noise reduction proximate the tissue contacting electrodes. Similarly, far-field signals including noise or artifacts can be reduced or canceled out for the overall end effector with a reference electrode that is not in contact with tissues and only with blood. Irrigation can be provided with irrigation ports 163a on one side and 162b on the other side in fluid communication with an irrigation line not shown disposed in a catheter shaft 90. Instead of an irrigation line separate from the catheter shaft 90, a lumen can be formed via extrusion of the catheter shaft 90 to provide for a lumen channel. It is noted that port 163a or 162b can be configured to have a sufficient flow diverter characteristic for irrigation fluid to cover the mapping electrodes during irrigation flow so as to prevent or reduce thrombus formations.

The flexible circuit 110 can further include a framework 130 contiguous to the insulative material or in the insulative material. In examples in which the flexible circuit 110 includes framework 130, the framework 130 can be disposed directly on the flexible circuit 110 with none, or very little, of the insulative material 120 coming between the two.

Stated otherwise, an aspect of the present disclosure provides an end effector 100 having a planar framework 130 bisecting two flat, heat formed portions 120a, 120b of a flexible insulating mass 120, with at least one flexible circuit 110 disposed on one side of the framework 130 and with contact surfaces 112c of electrodes 112 extending up to or slightly past the outer face of the flexible insulating mass 120.

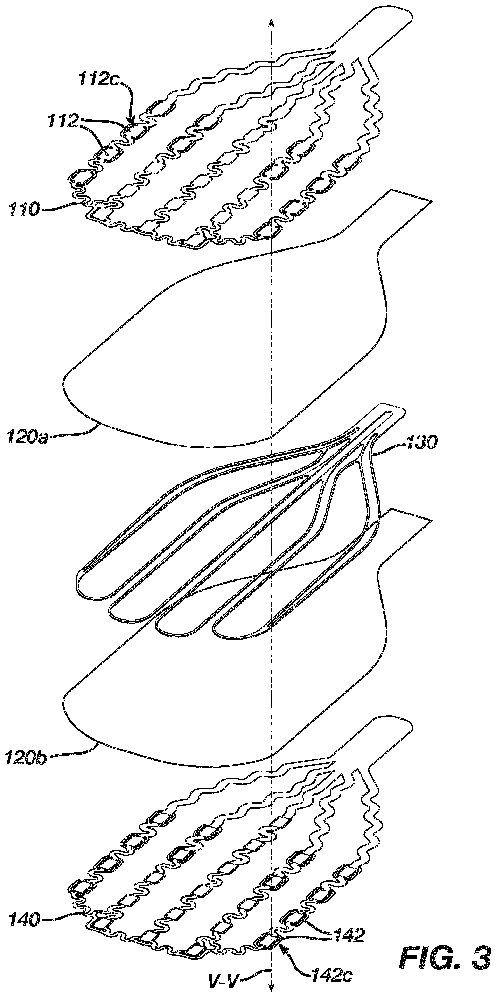

FIG. 3 shows an exploded view of the end effector 100, with the components thereof exploded vertically along vertical axis V-V. The flexible circuit 110 can be a first flexible circuit 110 and the plurality of electrodes 112 can be a first plurality of electrodes 112, with each first electrode 112 including a first contact surface 112c. The end effector 100 can further include a second flexible circuit 140 having a second plurality of electrodes 142. The second flexible circuit 140 can be spaced apart from the first flexible circuit 110 and each electrode of the second plurality of electrodes 142 can have a second contact surface 142c.

In examples having a first 110 and second 140 flexible circuits, the insulative material 120 can be disposed between the first flexible circuit 110 and the second flexible circuit 140, and the insulative material 120 can be contiguous to the second contact surfaces 142c so that only the contact surface 142c of each second electrode 142 is exposed to the ambient environment, similar to how first electrodes 112 are disposed in and exposed through the insulative material.

Electrodes 112, 142 can sense tissue signals or transmit energy AC or DC from an energy generator to the tissues. In some examples, there are about 92 electrodes. In some examples, there are about 48 electrodes. In some examples, there are about 64 electrodes. In some examples, there are about 72 electrodes. In some examples, there are about 98 electrodes. Details of the spacing of the electrodes for each pair vs spacing between discrete sets of pairs of electrodes can be found in US Provisional Patent Application S.N. 63/406,673 (Attorney Docket No. BIO6749USPSP3) filed on September 14, 2022 and included in the Appendix for incorporation by reference.

In examples wherein the end effector 100 includes a framework 130 disposed between the first flexible circuit 110 and the second flexible circuit 140.

Framework 130 can be a component of the end effector 100 that is separate and distinct from the first flexible circuit 110 and disposed proximate the first flexible circuit 110. In this case, the insulative material 120 can be further disposed between the framework 130 and the second flexible circuit 140. The framework 130 can be formed from a planar or cylindrical stock of material using any suitable method. For example, the framework 130 can be formed by cutting, laser cutting, stamping, etc.

Insulative material 120 can include a first sheet of insulative material 120a and a second sheet of insulative material 120b fused together proximate the framework 130 into a single, contiguous, generally planar insulative mass 120. This insulative material 120 also serves to enhance the atraumaticity of the end effector 100 and to protect the subject from sharp edges. The insulative material 120 can include polymer. As discussed later in relation to FIGs. 9A and 9B, the insulative material 120 can be heat formed around at least a portion of the first flexible circuit 110, the second flexible circuit 140, and the framework 130. The polymer can include thermoplastic polyurethane (TPU) or other heat formed or shaped material which lends itself to said heat forming.

Furthermore, while the insulative material 120 is shown to be flat in these figures, insulative material 120 can be shaped, scalloped, ribbed, ridged, concaved, convexed, or otherwise configured such that the overall profile of insulative material 120 yields physical and/or mechanical properties, such as rigidity and flexion along multiple axes, required by the end effector 100, mentioned above.

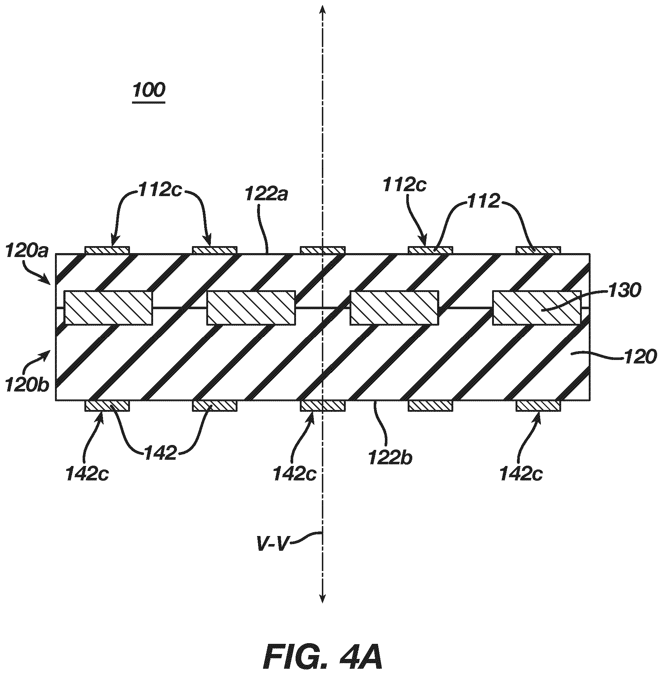

FIG. 4A shows a cross-section taken as indicated in FIG. 3. The first contact surfaces 112c lie substantially parallel to a first outer surface 122a of the insulative mass 120. The first plurality of electrodes 112 extending vertically and outwardly a first distance D₁ therefrom. Similarly, the second contact surfaces 142c can lie substantially parallel to a second outer surface 122b of the insulative mass 120 and extend substantially vertically and outwardly a second distance D₂ therefrom. As will be discussed later in relation to the fixture 500 shown in FIG. 5A-7B and FIGs. 9A-9B, first distance D₁ and second distance D₂ can be determined by aspects of fixture 500 and/or method 900.

FIG. 4B shows a similar cross-section, but from an example end effector 100 which does not have the second flexible circuit 140. That is, the end effector in Fig. 4B has electrodes on only one side (single sided end effector) as compared to an end effector of Fig. 4A with electrodes on opposite sides (or double sided end effector). FIG. 4B1 shows a variation of Fig. 4B in which the framework 130 is not encapsulated in the insulative material but disposed outside the insulative material 120.

FIG. 4C shows a cross-section similar to that of 4A, but from an example end effector 100 which does not have the framework 130. It should be noted that while framework 130 is shown in cross-section as rectangular, the framework 130 is not limited such cross-section and any suitable cross-sections can be utilized.

Importantly, the end effector 100 having flush or outwardly protruding electrode contact surfaces 112c, 142c can be manufactured without the need to remove material in order expose electrode contact surfaces 112c, 142c via the method and/or fixture described in more detail below.

In other examples, the first contact surfaces 112c can lie substantially coplanar to a first outer surface 122a of the insulative mass 120, and the second contact surfaces 142c lying coplanar to a second outer surface 122b of the insulative mass 120.

FIGs. 5A-6A show a fixture 500 for forming an encapsulated end effector for a medical catheter, such as the end effectors 100 of FIGs. 2-4C. It should be noted that the components to be formed into end effector 100 by fixture 500 are ordered FIG. 5A similar to those in FIG. 3. The first compression surface 510, the second compression surface 520, and side walls 550 are configured, when in the expanded configuration 500a, to accommodate therebetween: first flexible circuit 110, first sheet of insulative material 120a, a framework 130, a second sheet of insulative material 120b, and the second flexible circuit 140.

The fixture 500 can include a plurality of components stacked generally parallel. The plurality of components can include a first compression surface 510 and a second compression surface 520 disposed opposite the first compression surface 510 and configured to translate vertically with respect to the first compression surface 510 from an expanded configuration 500a to a compressed configuration 500b.

Fixture 500 can include a first flexible heat resistant sheet 530 disposed proximate the first compression surface 510.

In the detail view shown in FIG. 5B, electrode 112 is not protruding into the first flexible heat resistant sheet 530 because first compression surface 510 and second compression surface 520 are not being compressed toward one another, i.e., fixture 500 is in expanded configuration 500a.

The plurality of components can further include a second flexible heat resistant sheet 540 similar to the first flexible heat resistant sheet 530. Second flexible heat resistant sheet 540 can be disposed proximate the second compression surface 520.

In FIGs. 5A-5C, first sheet of insulative material 120a and second sheet of insulative material 120b are generally flat since they have not been molded yet. The first flexible heat resistant sheet 530 covers the contact surfaces 112c of electrodes 112 similar to a masking process. Following molding, when the contact surfaces 112c are no longer compressed into the first flexible heat resistant sheet 530, the contact surfaces 112c are exposed to the ambient environment, while the rest of the end effector 100 is encapsulated in material 120. As used herein, the term "molding" includes molding using heat, molding with pressure or molding with both heat and pressure.

Put simply, fixture 500 is a mold with at least one flexible portion 540 configured to allow electrode contact surfaces 112c to protrude into the flexible portion 540 so that the electrode contact surfaces 112c are not covered by the insulating material that is sandwiched between compression plates 510, 520 and over components of an end effector 100 including at least a first flexible circuit 110 and can include a portion of the electrodes 112. Fixture 500 can further include another flexible portion 540 that serves the same function for second electrode contact surfaces 142c of a second flexible circuit 140.

FIG. 6A shows compressed configuration 500b. The first compression surface 510 is configured, when in the compressed configuration 500b, to cause the first sheet of insulative material 120a to flow at least partially around the framework 130 and the first flexible circuit 110 extending up to approximately before the contact surfaces 112c the first plurality of electrodes 112. Side walls 550 are configured to constrain the first compression surface 510 as the fixture 500 is translated from the expanded 500a to the compressed 500b configuration. As seen in the detail view shown in FIG. 6B, first flexible heat resistant sheet 530 can have a durometer such that contact surfaces 112c of a first plurality of electrodes 112 extending from first flexible circuit 110 protrude a first distance D₁ into the first flexible heat resistant sheet 530 upon applying compression to the first compression surface 510 and the second compression surface 520. Similarly, in FIG. 6C, second flexible heat resistant sheet 540 can have a durometer such that contact surfaces 142c of a second plurality of electrodes 142 extending from a second flexible circuit 140 protrude into the second flexible heat resistant sheet 540 a second distance D₂ upon applying the compression.

In contrast to FIGs. 5A-5C, first sheet of insulative material 120a and second sheet of insulative material 120b shown in FIGs. 6A-6C have been molded and thus are no longer flat but rather form an encapsulating mass 120 of material in which the first sheet and the second sheet cannot necessarily be distinguished from one another. It is noted that while top electrode 112 and 142 are shown by itself, an alternative embodiment may have flexible printed circuit substrate 110 as part of the top electrode 112 and flexible printed circuit substrate 140 as part of bottom electrode 142 (Figs. 5B and 5C).

While first compression surface 510 and the second compression surface 520 are shown in the figures as being generally flat, surfaces 510, 520 can be shaped, scalloped, ribbed, ridged, concaved, convexed, or otherwise configured such that the overall profile of encapsulating mass 120 yields physical and/or mechanical properties, such as rigidity and flexion along multiple axes, required by the end effector 100, mentioned above.

Stated otherwise, the first compression surface 510 and the second compression surface 520 can be configured, when in the compressed configuration 500b, to cause the first sheet of insulative material 120a and the second sheet of insulative material 120b to fuse proximate the framework 130 and to flow at least partially around the framework 130, the first flexible circuit 110, and the second flexible circuit 140 extending up to approximately before the contact surfaces 112c of the first plurality of electrodes 112 and the contact surfaces of the second plurality of electrodes 142.

In some examples, the first distance D₁ can be equal to the second distance D₂. In some examples, the first distance D₁ can be approximately equal to 5 micrometers. The second distance D₂ can be approximately equal to 5 micrometers.

In examples in which fixture 500 does not include heat resistant sheets 530, 520, the electrode contact surfaces 112c, 142c can be placed directly in contact with the first compression surface 510 and the second compression surface 520. This results in the first contact surfaces 112c being substantially coplanar to first outer surface 122a of the insulative mass 120, and the second contact surfaces 142c being coplanar to second outer surface 122b of the insulative mass 120.

Fixture 500 can further include a heating element (not shown) configured to apply heat to the first sheet of insulative material 120a and the second sheet of insulative material 120b in order to heat-form them into the contiguous mass 120 discussed above. As previously mentioned, thermoplastic elastomers such as TPU can be used. While the first sheet of insulative material 120a and the second sheet of insulative material 120b fuse into a contiguous mass 120, this mass 120 is not necessarily homogenous. Characteristics of the finished end effector 100 produced with fixture 500 can be altered based on differences in composition between first sheet of insulative material 120a and the second sheet of insulative material 120b or additives placed therebetween and/or on the fixture 500.

First flexible heat resistant sheet 530 and second flexible heat resistant sheet 540 can be made of a material having properties enabling it to remain stable at temperatures which cause insulative material 120 to melt/flow. Furthermore, heat resistant sheets 530, 520 can include material properties that facilitate easy release from fixture 500. Fixture 500 can be configured to facilitate easy replacement of heat resistant sheets 530, 520, as they may be subject to more wear than other components of fixture 500.

Referring to Fig. 6A, application of heat or pressure (indicated by symbol "F"), the insulative material 120a and 120b begins to meld together as a single material 120 which flows around electrodes 112 and 142. As the top compression surface 510 and bottom compression surface 520 come together and make contact on respective top electrodes 112 and bottom electrodes 142, the insulative material 120 cannot flow around the electrode contact surfaces 112c and 142c. That is, the insulative material 120 cannot extend beyond the boundary defined by the top and bottom compression surfaces 510 and 520. The product after the heat or pressure molding is shown in Fig. 6D in which the insulative material 120 is now flush with the contact surface 112c (of top electrodes 112) and flush with the contact surfaces 142c of the bottom electrodes 142. While the preferred embodiments have the electrode contact surface 112c or 142c flush with the insulative surface 121a or 121b, in certain applications the contact surfaces do not have to be flush as shown in Figs. 6E and 6F in which the electrode 114 can protrude beyond the insulative surface 121a at varying levels of protrusions such as a low protrusion for electrodes 113 and high protrusion for electrodes 114 of Fig. 6E. In Fig. 6E, the varying level of protrusions is defined by the compression member 510' and 520' having the recessed pockets 512 and 514.

In applications where the electrode contact surfaces are to extend beyond a plane 121a or 121b defined by the insulative material 120, the top and bottom compression surfaces 510' and 520' can be provided with recessed openings 512 and 514 that mate directly to the respective electrodes such that the electrodes 112 and 142 would protrude for a predetermined distance above the top surface 121a and bottom surface 121b of the insulative material 120, shown here in Fig. 6E. In Fig. 6E, it can be seen that different protrusion heights can be obtained by varying the mating recessed openings in the compression surfaces 510' and 520' such that electrode 114 extends upper most above the top surface 121a of insulative material 120. Likewise, the recessed pockets 514 of compression plate 520' would allow for protrusion of electrodes 142 to extend further than the top or bottom surfaces 121a or 121b of insulative material 120.

Fig. 6F shows yet another embodiment within the scope of the present invention in that the electrode contact surfaces can be deeply recessed below the top or bottom surface 121a such as shown by electrode 112; flush with the top or bottom surface 121a as in electrode 113; protrudes beyond the top surface 121a and electrode 113 flush with the top surface 121a. While the top and bottom surfaces 121a and 121b are shown as flat or planar surfaces, it is within the scope of the present invention that the top and bottom surfaces of the insulative material 120 do not have to be flat or planar and may include compound curved or curved surfaces depending on the actual geometry of the compression surface 511 of plate 510. In all these examples, it should be mentioned that the contact surface of the electrode is not limited to its top surface but any surface of the electrode not covered by the insulative material.

FIG. 7A shows a perspective view of first compression surface 510, configured as a cap with a lip that serves as a stop, preventing compression past the compressed configuration 500b.

FIG. 7B shows a perspective view of fixture 500 with a finished end effector therein. In this figure, there is a Teflon sheet 502 configured to allow for release of the finished end effector, however, liquid, spray, or powdered release agents can be used. Fixture 500 can include notches, dovetails, keys, injection/exhaust ports, frames, plates, clamps, and other components used in molds as appreciated by those skilled in the pertinent art.

The present disclosure provides a catheter assembly 200 as shown in FIG. 8 which can include a tubular member 230 extending along a longitudinal axis L-L and configured to deliver end effector 100 to an out of a sheath 210. A physician 24 can manipulate the catheter 200 with handle 220. Appropriate examples for catheter assembly 200 and its subcomponents such as handle 220, sheath 210, tubular member 230, and others not mentioned herein are described in US Patent publication No. 2021/0369339, which is incorporated herein by reference and included in the Appendix attached hereto.

FIGs. 9A-9B show a method 900 of manufacturing an end effector for a medical catheter. Method 900 can in some examples be carried out using fixture 500 and can manufacture end effector 100 as detailed above, with all of their components and subcomponents.

As shown in FIG. 9A, method 900 can include placing 902 a flexible circuit including a plurality of electrodes in a mold, placing 904 a sheet of insulative material in contact with the flexible circuit, and molding 912 the flexible circuit and the insulative material to cause the insulative material to flow at least partially around the flexible circuit contiguous to the contact surface so that only the contact surface of at least a portion of the electrodes of the plurality of electrodes is exposed to the ambient environment.

The method 900 can further include, prior to molding placing 906a a second sheet of insulative material in contact with the first flexible circuit or the sheet of insulative material, placing 908a a second flexible circuit on the second sheet of insulative material opposite the first flexible circuit, the second flexible circuit comprising a second plurality of electrodes and each electrode of the plurality of electrodes comprising a second contact surface. The method 900 can further include placing 910a a second molding surface in contact with the second contact surfaces of the second plurality of electrodes. The method can include molding 914a the second flexible circuit and the second insulative material to cause the second insulative material to flow at least partially around the second flexible circuit, the insulative material extending up to approximately before the second contact surface.

Molding 912 the first sheet of insulative material and molding 914a the second sheet of insulative material can include applying heat to the first sheet of insulative material and the second sheet of insulative material, such as by the heating element of fixture 500 previously mentioned. Applying heat can include heating the first sheet of insulative material and heating the second sheet of insulative material to approximately 400 degrees Fahrenheit, which is sufficient to induce TPU to flow within fixture 500. As will be appreciated by one of skill in the art, the target temperature for heating insulative material can vary depending on the particular material.

Molding 912 the first sheet of insulative material and molding 914a the second sheet of insulative material can include fusing the first sheet of insulative material to the second sheet of insulative material proximate the first and second flexible circuits. This can result in contiguous mass 120. Molding 912 the first sheet of insulative material and molding 914a the second sheet of insulative material can include applying heat and compression to the first contact surfaces of the first plurality of electrodes and the second contact surfaces of the second plurality of electrodes, such as by first 510 and second 520 compression surfaces of fixture 500.

As shown in FIG. 9B, method 900 can include placing 902 a flexible circuit including a plurality of electrodes in a mold, placing 904 a sheet of insulative material in contact with the flexible circuit, 905 a framework in contact with the sheet of insulative material, and molding 912 the flexible circuit and the insulative material to cause the insulative material to flow at least partially around the flexible circuit and the framework contiguous to the contact surface so that only the contact surface of at least a portion of the electrodes of the plurality of electrodes is exposed to the ambient environment.

The method 900 can further include, prior to molding, placing 906b a second sheet of insulative material in contact with the framework, placing 908b a second flexible circuit on the second sheet of insulative material opposite the framework, the second flexible circuit comprising a second plurality of electrodes and each electrode of the plurality of electrodes comprising a second contact surface. The method 900 can further include placing 910b a second molding surface in contact with the second contact surfaces of the second plurality of electrodes. The method can include molding 914b the second flexible circuit, the second insulative material, and the framework to cause the second insulative material to flow around the framework and at least partially around the second flexible circuit, the insulative material extending up to approximately before the second contact surface.

Molding 912 the first sheet of insulative material and molding 914b the second sheet of insulative material can include applying heat to the first sheet of insulative material and the second sheet of insulative material, such as by the heating element of fixture 500 previously mentioned. Applying heat can include heating the first sheet of insulative material and heating the second sheet of insulative material to approximately 400 degrees Fahrenheit, which is sufficient to induce TPU to flow within fixture 500. As will be appreciated by one of skill in the art, the target temperature for heating insulative material can vary depending on the particular material.

Molding 912 the first sheet of insulative material and molding 914b the second sheet of insulative material can include fusing the first sheet of insulative material to the second sheet of insulative material proximate the framework. This can result in contiguous mass 120. Molding 912 the first sheet of insulative material and molding 914b the second sheet of insulative material can include applying heat and compression to the first contact surfaces of the first plurality of electrodes and the second contact surfaces of the second plurality of electrodes, such as by first 510 and second 520 compression surfaces of fixture 500.

In other terms, the process 900 for making the end effector includes protecting electrode contact surfaces by allowing them to squish into a flexible sheet, then molding flexible insulating material over components of the end effector, for example a flexible circuit with the electrodes disposed thereon and a framework.

It should be appreciated that the method 900 just described can include more or fewer steps or features than those described. Furthermore, the steps or features can be performed in different orders and should not be construed as being required to be performed in any particular order.

The disclosed technology described herein can be further understood according to the following clauses:
Clause 1: An end effector comprising: a flexible circuit comprising a plurality of electrodes, the flexible circuit disposed on an insulative material and each electrode of the plurality of electrodes comprising a contact surface; and the insulative material being contiguous to the contact surface so that only the contact surfaces of at least a portion of the plurality of electrodes are exposed to the ambient environment.
Clause 2: The end effector of clause 1, the flexible circuit further comprising a framework contiguous to the insulative material or with a portion of the framework encapsulated within the insulative material and wherein the contact surface of the electrode comprises area of the contact electrode not covered by the insulative material.
Clause 3: The end effector of clause 1, the flexible circuit comprising a first flexible circuit and the plurality of electrodes comprising a first plurality of electrodes each comprising a first contact surface, the end effector further comprising: a second flexible circuit comprising a second plurality of electrodes, the second flexible circuit being spaced apart from the first flexible circuit and each electrode of the second plurality of electrodes comprising a second contact surface, the insulative material being further disposed between the first flexible circuit and the second flexible circuit, the insulative material being contiguous to the second contact surfaces so that only the contact surface of each electrode is exposed to the ambient environment.
Clause 4: The end effector of clause 3, the end effector further comprising a framework disposed between the first flexible circuit and the second flexible circuit.
Clause 5: The end effector of any of clauses 3-4, the insulative material being heat formed around at least a portion of the first flexible circuit, the second flexible circuit, and the framework.
Clause 6: The end effector of clause 1, the flexible circuit comprising a first flexible circuit and the plurality of electrodes comprising a first plurality of electrodes each comprising a first contact surface, the end effector further comprising: a framework disposed proximate the first flexible circuit; a second flexible circuit comprising a second plurality of electrodes, the second flexible circuit being spaced apart from the framework on a side of the framework opposite the first flexible circuit and each electrode of the second plurality of electrodes comprising a second contact surface, the insulative material being further disposed between the framework and the second flexible circuit, the insulative material being heat formed around the framework and at least a portion of the second flexible circuit.
Clause 7: The end effector of any of clauses 2-6, the insulative material comprising a first sheet of insulative material and a second sheet of insulative material fused together proximate the framework into a single, contiguous, generally planar insulative mass.
Clause 8: The end effector of clause 7, the first contact surfaces lying parallel to a first outer surface of the insulative mass, the first plurality of electrodes extending vertically and outwardly a first distance therefrom.
Clause 9: The end effector of clause 7, the first contact surfaces lying coplanar to a first outer surface of the insulative mass.
Clause 10: The end effector of any of clauses 7-9, the second contact surfaces lying substantially parallel to a second outer surface of the insulative mass, the second plurality of electrodes extending substantially vertically and outwardly a second distance therefrom.
Clause 11: The end effector of any of clauses 7-9, the second contact surfaces lying coplanar to a second outer surface of the insulative mass.
Clause 12: The end effector of any of clauses 1-11, the insulative material comprising POLYMER.
Clause 13: The end effector of clause 12, wherein the polymer comprises thermoplastic polyurethane (TPU).
Clause 14: A method of manufacturing an end effector for a medical catheter, the method comprising: placing a flexible circuit comprising a plurality of electrodes in a mold, each electrode of the plurality of electrodes comprising a contact surface; placing a sheet of insulative material in contact with the flexible circuit; and molding the flexible circuit and the insulative material to cause the insulative material to flow at least partially around the flexible circuit contiguous to the contact surface so that only the contact surface of at least a portion of the electrodes of the plurality of electrodes is exposed to the ambient environment.
Clause 15: The method of clause 14, the sheet of insulative material comprising a first sheet of flexible material, the flexible circuit comprising a first flexible circuit and comprising a framework, and the plurality of electrodes comprising a first plurality of electrodes comprising first contact surfaces, the method further comprising, prior to the molding: placing a second sheet of insulative material in contact with the framework; placing a second flexible circuit on the second sheet of insulative material opposite the framework, the second flexible circuit comprising a second plurality of electrodes, each electrode of the plurality of electrodes comprising a second contact surface; placing a second molding surface in contact with the second contact surfaces of the second plurality of electrodes; and molding the second flexible circuit, the second insulative material, and the framework to cause the second insulative material to flow around the framework and at least partially around the second flexible circuit, the insulative material extending up to approximately before the second contact surface.
Clause 16: The method of clause 14, the sheet of insulative material comprising a first sheet of flexible material, the flexible circuit comprising a first flexible circuit and comprising a framework, and the plurality of electrodes comprising a first plurality of electrodes comprising first contact surfaces, the method further comprising, prior to the molding: placing a second sheet of insulative material in contact with the framework; placing a second flexible circuit on the second sheet of insulative material opposite the framework, the second flexible circuit comprising a second plurality of electrodes, each electrode of the plurality of electrodes comprising a second contact surface; placing a second molding surface in contact with the second contact surfaces of the second plurality of electrodes; and molding the second flexible circuit, the second insulative material, and the framework to cause the second insulative material to flow around the framework and at least partially around the second flexible circuit, the insulative material extending up to approximately before the second contact surface.
Clause 17: The method of any of clauses 15-16, wherein molding the first sheet of insulative material and molding the second sheet of insulative material comprise applying heat to the first sheet of insulative material and the second sheet of insulative material.
Clause 18: The method of clause 17, applying heat comprising heating the first sheet of insulative material and heating the second sheet of insulative material to approximately 400 degrees Fahrenheit.
Clause 19: The method of any of clauses 15-18, molding the first sheet of insulative material and molding the second sheet of insulative material comprising fusing the first sheet of insulative material to the second sheet of insulative material proximate the framework.
Clause 20: The method of clause any of clauses 17-19, the first sheet of insulative material and the second sheet of insulative material comprising a thermoplastic elastomer, and wherein molding the first sheet of insulative material and molding the second sheet of insulative material comprises applying heat and compression to the first contact surfaces of the first plurality of electrodes and the second contact surfaces of the second plurality of electrodes.
Clause 21: The method of any of clauses 15-20, the first molding surface comprising a first flexible heat resistant sheet configured to conform to each of the contact surfaces of the first plurality of electrodes, and the second molding surface comprising a second flexible heat resistant sheet configured to conform to each of the contact surfaces of the second plurality of electrodes.
Clause 22: The method of clause 21, wherein the first flexible heat resistant sheet comprises a material having a durometer such that the first plurality of electrodes extend into the first flexible heat resistant sheet a first distance upon applying the compression.
Clause 23: The method of clause 22, wherein the first distance comprises approximately 5 micrometers.
Clause 24: The method of any of clauses 21-23, wherein the second flexible heat resistant sheet comprises a material having a durometer such that the second plurality of electrodes extend into the second flexible heat resistant sheet a second distance upon applying the compression.
Clause 25: The method of clause 24, wherein the second distance comprises approximately 5 micrometers.
Clause 26: A fixture for forming an encapsulated end effector for a medical catheter, the fixture comprising a plurality of components stacked generally parallel along a vertical axis of the fixture, the plurality of components comprising: a first compression surface; a second compression surface disposed opposite the first compression surface and configured to translate along the vertical axis with respect to the first compression surface from an expanded configuration to a compressed configuration; and a first flexible heat resistant sheet disposed proximate the first compression surface and comprising a durometer such that contact surfaces of a first plurality of electrodes extending from a first flexible circuit protrude into the first flexible heat resistant sheet a first distance upon applying compression to the first compression surface and the second compression surface.
Clause 27: The fixture of clause 26, the plurality of components further comprising: a second flexible heat resistant sheet disposed proximate the second compression surface and comprising a durometer such that contact surfaces a second plurality of electrodes extending from a second flexible circuit protrude into the second flexible heat resistant sheet a second distance upon applying the compression.
Clause 28: The fixture of clause 27, the first compression surface and the second compression surface configured, when in the expanded configuration, to accommodate therebetween: the first flexible circuit, a first sheet of insulative material, a framework, a second sheet of insulative material, and the second flexible circuit.
Clause 29: The fixture of any of clauses 26-28, the first compression surface configured, when in the compressed configuration, to cause the first sheet of insulative material to flow at least partially around the framework and the first flexible circuit extending up to approximately before the contact surfaces the first plurality of electrodes.
Clause 30: The fixture of any of clauses 27-29, the first compression surface and the second compression surface configured, when in the compressed configuration, to cause the first sheet of insulative material and the second sheet of insulative material to fuse proximate the framework and to flow at least partially around the framework, the first flexible circuit, and the second flexible circuit extending up to approximately before the contact surfaces of the first plurality of electrodes and the contact surfaces of the second plurality of electrodes.
Clause 31: The fixture of any of clauses 27-30, the first distance being equal to the second distance.
Clause 32: The fixture of any of clauses 26-31, the first distance being approximately equal to 5 micrometers.
Clause 33: The fixture of any of clauses 27-32, the second distance being approximately equal to 5 micrometers.
Clause 34: The fixture of any of clauses 27-33, further comprising a heating element configured to apply heat to the first sheet of insulative material and the second sheet of insulative material.

## Claims

1. An end effector comprising:
a flexible circuit comprising a plurality of electrodes, the flexible circuit disposed on an insulative material and each electrode of the plurality of electrodes comprising a contact surface; and
the insulative material being contiguous to the contact surface so that only the contact surfaces of at least a portion of the plurality of electrodes are exposed to an ambient environment.

2. The end effector of claim 1, the flexible circuit further comprising a framework contiguous to the insulative material or with a portion of the framework encapsulated within the insulative material and wherein the contact surface of an electrode of the plurality of electrodes comprises area of the electrode not covered by the insulative material.

3. The end effector of claim 2, the flexible circuit comprising a first flexible circuit and the plurality of electrodes comprising a first plurality of electrodes each comprising a first contact surface, the end effector further comprising:
a second flexible circuit comprising a second plurality of electrodes, the second flexible circuit being spaced apart from the first flexible circuit and each electrode of the second plurality of electrodes comprising a second contact surface, the insulative material being further disposed between the first flexible circuit and the second flexible circuit, the insulative material being contiguous to the second contact surfaces so that only the contact surface of each electrode is exposed to the ambient environment.

4. The end effector of claim 3, the end effector further comprising a framework disposed between the first flexible circuit and the second flexible circuit, and/or the insulative material being heat formed around at least a portion of the first flexible circuit, the second flexible circuit, and the framework.

5. The end effector of claim 1, the flexible circuit comprising a first flexible circuit and the plurality of electrodes comprising a first plurality of electrodes each comprising a first contact surface, the end effector further comprising:
a framework disposed proximate the first flexible circuit;
a second flexible circuit comprising a second plurality of electrodes, the second flexible circuit being spaced apart from the framework on a side of the framework opposite the first flexible circuit and each electrode of the second plurality of electrodes comprising a second contact surface, the insulative material being further disposed between the framework and the second flexible circuit, the insulative material being heat formed around the framework and at least a portion of the second flexible circuit.

6. The end effector of any of claims 2 to 5, the insulative material comprising a first sheet of insulative material and a second sheet of insulative material fused together proximate the framework into a single, contiguous, generally planar insulative mass, optionally the first contact surfaces lying parallel to a first outer surface of the insulative mass, the first plurality of electrodes extending vertically and outwardly a first distance therefrom.

7. A method of manufacturing an end effector for a medical catheter, the method comprising:
placing a flexible circuit comprising a plurality of electrodes in a mold, each electrode of the plurality of electrodes comprising a contact surface;
placing a sheet of insulative material in contact with the flexible circuit; and
molding the flexible circuit and the insulative material to cause the insulative material to flow at least partially around the flexible circuit contiguous to the contact surface so that only the contact surface of at least a portion of the electrodes of the plurality of electrodes is exposed to an ambient environment.

8. The method of claim 7, the sheet of insulative material comprising a first sheet of flexible material, the flexible circuit comprising a first flexible circuit, and the plurality of electrodes comprising a first plurality of electrodes comprising first contact surfaces, the method further comprising, prior to the molding:
placing a second sheet of insulative material in contact with the first flexible circuit;
placing a second flexible circuit on the second sheet of insulative material opposite the first flexible circuit, the second flexible circuit comprising a second plurality of electrodes, each electrode of the plurality of electrodes comprising a second contact surface;
placing a second molding surface in contact with the second contact surfaces of the second plurality of electrodes; and
molding the second flexible circuit and the second insulative material to cause the second insulative material to flow at least partially around the second flexible circuit, the insulative material extending up to approximately before the second contact surface.

9. The method of claim 7, the sheet of insulative material comprising a first sheet of flexible material, the flexible circuit comprising a first flexible circuit and comprising a framework, and the plurality of electrodes comprising a first plurality of electrodes comprising first contact surfaces, the method further comprising, prior to the molding:
placing a second sheet of insulative material in contact with the framework;
placing a second flexible circuit on the second sheet of insulative material opposite the framework, the second flexible circuit comprising a second plurality of electrodes, each electrode of the plurality of electrodes comprising a second contact surface;
placing a second molding surface in contact with the second contact surfaces of the second plurality of electrodes; and
molding the second flexible circuit, the second insulative material, and the framework to cause the second insulative material to flow around the framework and at least partially around the second flexible circuit, the insulative material extending up to approximately before the second contact surface.

10. The method of claim 8 or claim 9, wherein molding the first sheet of insulative material and molding the second sheet of insulative material comprise applying heat to the first sheet of insulative material and the second sheet of insulative material, and optionally applying heat comprising heating the first sheet of insulative material and heating the second sheet of insulative material to approximately 400 degrees Fahrenheit.

11. The method of any of claims 8 to 10, molding the first sheet of insulative material and molding the second sheet of insulative material comprising fusing the first sheet of insulative material to the second sheet of insulative material proximate the framework.

12. The method of claim 10 or claim 11, the first sheet of insulative material and the second sheet of insulative material comprising a thermoplastic elastomer, and
wherein molding the first sheet of insulative material and molding the second sheet of insulative material comprises applying heat and compression to the first contact surfaces of the first plurality of electrodes and the second contact surfaces of the second plurality of electrodes.

13. The method of any of claims 10 to 12, the first molding surface comprising a first flexible heat resistant sheet configured to conform to each of the contact surfaces of the first plurality of electrodes, and
the second molding surface comprising a second flexible heat resistant sheet configured to conform to each of the contact surfaces of the second plurality of electrodes, optionally wherein the first flexible heat resistant sheet comprises a material having a durometer such that the first plurality of electrodes extend into the first flexible heat resistant sheet a first distance upon applying compression.

14. A fixture for forming an encapsulated end effector for a medical catheter, the fixture comprising a plurality of components stacked generally parallel along a vertical axis of the fixture, the plurality of components comprising:
a first compression surface;
a second compression surface disposed opposite the first compression surface and configured to translate along the vertical axis with respect to the first compression surface from an expanded configuration to a compressed configuration; and
a first flexible heat resistant sheet disposed proximate the first compression surface and comprising a durometer such that contact surfaces of a first plurality of electrodes extending from a first flexible circuit protrude into the first flexible heat resistant sheet a first distance upon applying compression to the first compression surface and the second compression surface.

15. The fixture of claim 14, the plurality of components further comprising:
a second flexible heat resistant sheet disposed proximate the second compression surface and comprising a durometer such that contact surfaces a second plurality of electrodes extending from a second flexible circuit protrude into the second flexible heat resistant sheet a second distance upon applying the compression, optionally the first compression surface and the second compression surface configured, when in the expanded configuration, to accommodate therebetween: the first flexible circuit, a first sheet of insulative material, a framework, a second sheet of insulative material, and the second flexible circuit.
